# EUROPÄISCHE PATENTANMELDUNG

(11) **EP 0 693 477 A1**
(43) Veröffentlichungstag der Anmeldung: **24.01.1996**
(21) Anmeldenummer: 95110777.0
(22) Anmeldetag: 11.07.1995
(51) Int. Cl.: C07D 213/82, C07D 213/83, C07D 409/12, C07D 413/04, C07D 409/04, A01N 43/40

(54) **Hydroxypyridoncarbonsäureamide, deren Herstellung und Verwendung**

(30) Priorität: 20.07.1994 DE 4425616
(71) Anmelder: BASF AKTIENGESELLSCHAFT, D-67056 Ludwigshafen (DE)
(72) Erfinder: von Deyn, Wolfgang, Dr., D-67434 Neustadt (DE); Kardorff, Uwe, Dr., D-68159 Mannheim (DE); Nübling, Christoph, Dr., D-67454 Hassloch (DE); Theobald, Hans, Dr., D-67117 Limburgerhof (DE); Kappe, Thomas, Prof. Dr., A-8010 Graz (AT); Walter, Helmut, Dr., D-67283 Obrigheim (DE); Gerber, Matthias, Dr., D-67117 Limburgerhof (DE); Westphalen, Karl-Otto, Dr., D-67346 Speyer (DE)

(57) **Zusammenfassung**

Hydroxypyridoncarbonsäureamide der Formel I 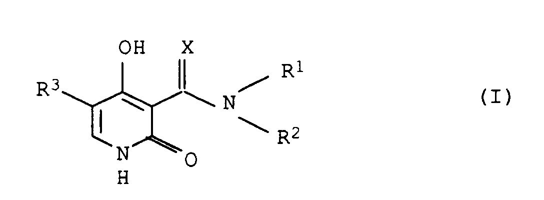
in der die Substituenten folgende Bedeutungen haben:
- X: Sauerstoff oder Schwefel;
- R¹: Wasserstoff, Alkyl, Alkenyl, Alkinyl, Cycloalkyl, Phenyl, wobei die organischen Gruppen substituiert sein können;
- R²: Wasserstoff, Hydroxyl, Alkoxy, Alkenyloxy, Dialkylamino, Alkyl, Alkenyl, Alkinyl, Cycloalkyl, wobei die organischen Gruppen substituiert sein können;
oder
- R¹, R²: gemeinsam eine Alkylenkette mit 4 bis 7 Gliedern, welche durch Sauerstoff, Schwefel oder N-Methyl unterbrochen sein kann;
- R³: Wasserstoff, Halogen, Nitro, Alkyl, Alkenyl, Alkinyl, Cycloalkyl, Arylalkyl, Alkoxy, Halogenalkoxy, Alkylthio, Halogenalkylthio, Di-alkylamino, Phenyl, Phenylthio, Phenoxy oder ein 5- oder 6-gliedriger heterocyclischer, aliphatischer oder aromatischer Rest, enthaltend ein bis drei Heteroatome, ausgewählt aus der Gruppe Sauerstoff, Schwefel oder Stickstoff, wobei die genannten aromatischen oder heteroaromatischen Reste gegebenenfalls substituiert sind.

## Beschreibung

Die Erfindung betrifft Hydroxypyridoncarbonsäureamide der allgemeinen Formel I 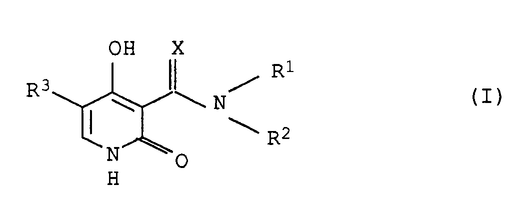
in der die Substituenten folgende Bedeutungen haben:
- X: Sauerstoff oder Schwefel;
- R¹: Wasserstoff, C₁-C₁₀-Alkyl, C₃-C₁₀-Alkenyl, C₃-C₁₀-Alkinyl, C₃-C₈-Cycloalkyl, Phenyl, wobei diese Gruppen ein bis fünf Halogenatome und/oder einen bis drei der folgenden Reste tragen können: Cyano, C₃-C₈-Cycloalkyl, C₁-C₄-Alkoxy, C₁-C₄-Halogenalkoxy, C₁-C₄-Alkylthio, C₁-C₄-Halogenalkylthio, Di-C₁-C₄-Alkylamino, Phenyl, Phenylthio, Phenoxy, wobei die Phenylreste ihrerseits eine bis drei der folgenden Gruppen tragen können: C₁-C₄-Alkyl, C₁-C₄-Alkoxy, C₁-C₄-Halogenalkyl,C₁-C₄-Halogenalkoxy, Halogen, Cyano oder Nitro;
- R²: Wasserstoff, Hydroxyl, C₁-C₈-Alkoxy, C₃-C₈-Alkenyloxy, Di-C₁-C₄-Alkylamino, C₁-C₁₀-Alkyl, C₃-C₁₀-Alkenyl, C₃-C₁₀-Alkinyl, C₃-C₈-Cycloalkyl, wobei die Gruppen ein bis fünf Halogenatome und/oder einen bis drei der folgenden Reste tragen können: Cyano, C₃-C₈-Cycloalkyl, C₁-C₄-Alkoxy, C₁-C₄-Halogenalkoxy, C₁-C₄-Alkylthio, C₁-C₄-Halogenalkylthio, Di-C₁-C₄-Alkylamino, Phenyl, Phenylthio, Phenoxy, wobei die Phenylreste ihrerseits eine bis drei der folgenden Gruppen tragen können: C₁-C₄-Alkyl, C₁-C₄-Alkoxy, C₁-C₄-Halogenalkyl, C₁-C₄-Halogenalkoxy, Halogen, Cyano oder Nitro;
- R²: ferner substituiertes C₁-C₈-Alkoxy, C₃-C₈-Alkenyloxy, Di-C₁-C₄-Alkylamino, C₁-C₁₀-Alkyl, C₃-C₁₀-Alkenyl, C₃-C₁₀-Alkinyl, C₃-C₈-Cycloalkyl, wobei diese Gruppen durch ein bis fünf Halogenatome und/oder ein bis zwei 5- bis 6-gliedrige heteroyclische, aliphatische oder aromatische Reste, enthaltend ein bis drei Heteroatome, ausgewählt aus der Gruppe Sauerstoff, Schwefel oder Stickstoff substituiert sind, die ein oder zwei der folgenden Substituenten tragen können: Halogen, C₁-C₄-Alkyl, C₁-C₄-Alkoxy, C₁-C₄-Halogenalkyl oder C₁-C₄-Halogenalkoxy;
oder
- R¹, R²: gemeinsam eine Alkylenkette mit 4 bis 7 Gliedern, welche durch Sauerstoff, Schwefel oder N-Methyl unterbrochen sein kann;
- R³: Wasserstoff, Halogen, Nitro, C₁-C₁₀-Alkyl, C₂-C₁₀-Alkenyl, C₂-C₁₀-Alkinyl, C₃-C₈-Cycloalkyl, C₇-C₁₅-Arylalkyl, C₁-C₆-Alkoxy, C₁-C₆-Halogenalkoxy, C₁-C₆-Alkylthio, C₁-C₆-Halogenalkylthio, Di-C₁-C₆-Alkylamino, Phenyl, Phenylthio, Phenoxy oder ein 5- oder 6-gliedriger heterocyclischer, aliphatischer oder aromatischer Rest, enthaltend ein bis drei Heteroatome, ausgewählt aus der Gruppe Sauerstoff, Schwefel oder Stickstoff, wobei die genannten aromatischen oder heteroaromatischen Reste gegebenenfalls substituiert sind durch Halogen, Cyano, Nitro, C₁-C₄-Alkyl, C₁-C₄-Halogenalkyl, C₃-C₈-Cycloalkyl, C₁-C₄-Alkoxy, C₁-C₄-Halogenalkoxy, C₁-C₄-Alkylthio, C₁-C₄-Halogenalkylthio oder Di-C₁-C₄-Alkylamino;
und deren umweltverträgliche Salze.

Außerdem betrifft die Erfindung Verfahren zur Herstellung dieser Verbindungen, ihre Verwendung als Herbizide und zur Regulierung des Pflanzenwachstums sowie herbizide Mittel, welche diese Verbindungen als wirksame Substanzen enthalten.

Herbizid wirksame Hydroxypyridoncarbonsäureamide sind bereits aus der EP-A 544 151 bekannt. Die dort beschriebenen Verbindungen tragen alle in der 6-Position des Pyridonrings einen Substituenten, z.B. einen Methylrest. An der 6-Position unsubstituierte Hydroxypyridon-3-carbonsäureamide sind bisher nicht bekannt.

Die herbiziden Eigenschaften sowie die Verträglichkeit gegenüber Kulturpflanzen der bekannten Verbindungen können jedoch nur bedingt befriedigen.

Aufgabe der vorliegenden Erfindung war es deshalb, neue Hydroxypyridoncarbonsäureamide mit verbesserten Eigenschaften bereitzustellen. Demgemäß wurden die eingangs beschriebenen Verbindungen, sowie Verfahren zu deren Herstellung und herbizide Mittel, die die Verbindungen I enthalten, gefunden.

Besonders bevorzugt sind Hydroxypyridoncarbonsäureamide mit X = Sauerstoff. Der Rest R¹ steht vorzugsweise für Wasserstoff.
- R²: bedeutet vorzugsweise Wasserstoff, C₁-C₁₀-Alkyl, C₃-C₁₀-Alkenyl, C₃-C₁₀-Alkinyl, C₃-C₈-Cycloalkyl, C₁-C₈-Alkoxy, wobei die genannten Reste durch Halogen, C₁-C₄-Alkoxy, C₁-C₄-Halogenalkoxy oder Phenyl substituiert sein können, wobei der Phenylrest seinerseits ein bis drei der folgenden Gruppen tragen kann:
C₁-C₄-Alkyl, C₁-C₄-Alkoxy, C₁-C₄-Halogenalkyl, Halogen oder C₁-C₄-Halogenalkoxy;
- R³: Wasserstoff, C₁-C₁₀-Alkyl, C₁-C₁₀-Halogenalkyl, C₂-C₁₀-Alkenyl, C₂-C₁₀-Halogenalkenyl, C₂-C₁₀-Alkinyl, C₃-C₁₀-Cycloalkyl, C₇-C₁₅-Arylalkyl oder Halogen.

Die Hydroxypyridoncarbonsäureamide I können in folgenden tautomeren Formeln vorliegen: 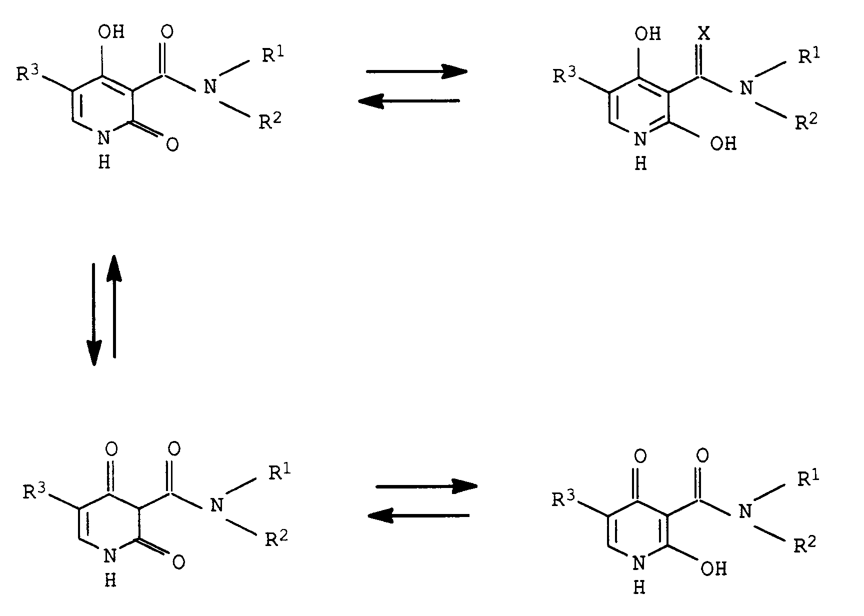
Die Herstellung der Hydroxypyridoncarbonsäureamide I gelingt durch Umsetzung von Verbindungen der Formel II, 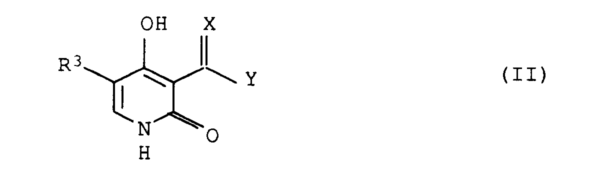
in der X und R³ die in Anspruch 1 angegebene Bedeutungen haben und Y ein Halogenatom, eine OH-Gruppe oder eine Alkoxygruppe bedeuten, mit einem Amin der Formel III, 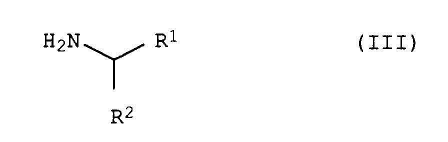
in der R¹ und R² die oben angegebenen Bedeutungen haben, unter geeigneten, allgemein bekannten Bedingungen.

Besonders vorteilhaft werden Alkylester IIa (Y = O-Alkyl), z.B. Methyl- oder Ethylester mit den Aminen der allgemeinen Formel III umgesetzt.

Diese Amidierung erfolgt im allgemeinen im Temperaturbereich von 50 - 250 °C, vorzugsweise 110 - 160 °C im Rührverfahren oder im Autoklaven.

Als Lösungsmittel eignen sich Alkohole wie Methanol, Ethanol, Propanol sowie Kohlenwasserstoffe wie Benzol oder Toluol, Wasser, Ether wie Diethylether oder Tetrahydrofuran, sowie aprotische dipolare Lösungsmittel wie Dimethylformamid, Dimethylsulfoxid oder Pyridin.

Es können auch Gemische dieser Stoffe als Lösungs- und Verdünnungsmittel verwendet werden.

Die Ausgangsstoffe werden üblicherweise in stöchiometrischen Mengen miteinander umgesetzt. Es kann, beispielsweise zur Steigerung der Ausbeute, vorteilhaft sein, einen der Ausgangsstoffe, vorzugsweise das Amin, in einem Überschuß, z.B. in einer Menge von 0,1 bis 10 Moläquivalenten, bezogen auf II zu verwenden.

Die Amidierung wird im allgemeinen bei Atmosphärendruck ausgeführt. Es kann jedoch, je nach Art des verwendeten Amins oder Lösungsmittels, vorteilhaft sein, die Umsetzung unter erhöhtem Druck, insbesondere unter autogen erhöhtem Druck in einem Autoklaven durchzuführen.

Die Ausgangsverbindungen der Formel IIa lassen sich nach allgemein bekannten chemischen Verfahren (J. Org. Chem. 51, 1374ff (1986)) aus Aminoacrylestern IV und Malonestern V herstellen.

Außerdem erhält man die Zwischenprodukte der allgemeinen Formel IIa durch thermische Cyclisierung der aus Vinylisocyanaten VI und Malonestern V erhaltenen Acylierungsprodukte VII nach bekannten chemischen Verfahren (J. Heterocyclic Chem., 22, 985ff (1985)). 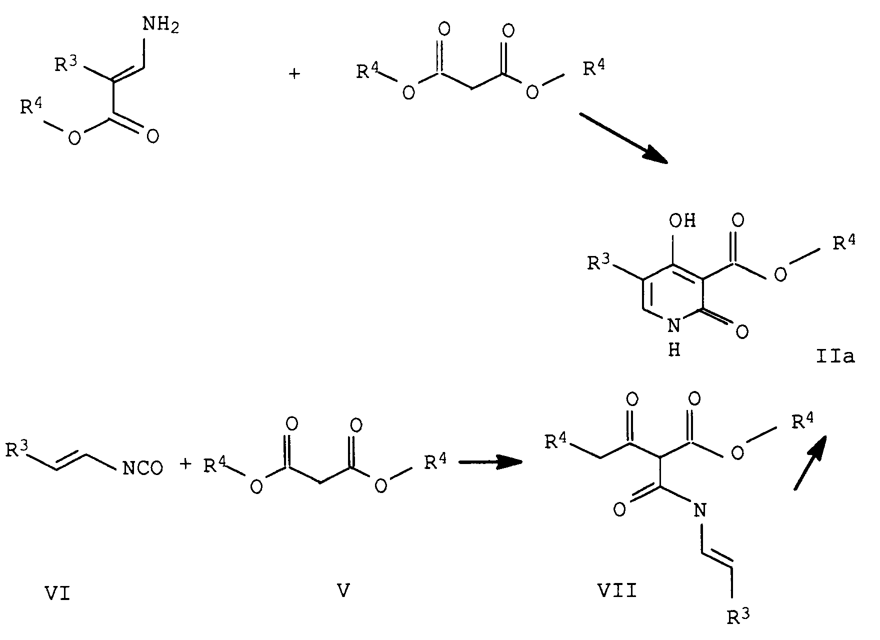
Die für die Umsetzungen benötigten Aminoacrylester IV sind bekannt oder lassen sich nach bekannten chemischen Verfahren (Annales de Chimie 18, 81ff (1932)) herstellen.

Die Vinylisocyanate VI sind ebenfalls bekannt oder lassen sich nach bekannten chemischen Verfahren (Angew. Chem. 91, 334ff (1979); J. Chem. Soc. Perkin Trans. I 2185ff (1988)) herstellen.

Die für die Umsetzungen benötigten Amine III und Malonester V sind bekannt, kommerziell erhältlich oder lassen sich nach allgemein bekannten chemischen Verfahren herstellen.

Aufgrund ihres sauren Charakters können die erfindungsgemäßen Hydroxypyridoncarbonsäureamide I Salze von Alkali- oder Erdalkalimetallverbindungen sowie Tetraalkylammoniumsalze, Benzyltrialkylammoniumsalze, Trialkylsulfoniumsalze oder Trialkylsulfoxoniumsalze bilden.

Alkalimetallsalze der Verbindungen I können durch Behandeln von I mit Natrium- oder Kaliumhydroxid oder -alkoholat in wäßriger Lösung oder in einem organischen Lösungsmittel wie Methanol, Ethanol, Aceton oder Toluol erhalten werden. Andere Metallsalze, z. B. die Mangan-, Kupfer-, Zink-, Eisen-, Calcium-, Magnesium- und Bariumsalze oder Salze mit organischen Kationen können aus den Natriumsalzen in üblicher Weise hergestellt werden.
- R⁴: in den Formel IIa, IV, V, und VII steht für C₁-C₆-Alkyl wie Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 1,1,2-Trimethylpropyl, 1,2,2-Trimethylpropyl, 1-Ethyl-1-methylpropyl oder 1-Ethyl-2-methyl-propyl, insbesondere Methyl und Ethyl.

Im Hinblick auf die bestimmungsgemäße Verwendung der Hydroxypyridoncarbonsäureamide der allgemeinen Formel I kommen als Substituenten folgende Reste in Betracht:
- R¹: Wasserstoff;
unverzweigtes oder verzweigtes C₁-C₁₀-Alkyl, wie Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 1,1,2-Trimethylpropyl, 1-Ethyl-1-methylpropyl, 1-Ethyl-2-methyl-propyl, Heptyl, 1-Methylheptyl, Octyl, Nonyl und Decyl
insbesondere C₁-C₆-Alkyl wie Methyl, Ethyl, 1-Methylethyl, 1-Methylpropyl, 2-Methylpropyl, 1,1-Dimethylethyl und 1,1-Dimethylpropyl;
unverzweigtes oder verzweigtes C₃-C₁₀-Alkenyl wie 2-Propenyl, 2-Butenyl, 3-Butenyl, 1-Methyl-2-propenyl, 2-Methyl-2-propenyl, 2-Pentenyl, 3-Pentenyl, 4-Pentenyl, 3-Methyl-2-butenyl, 1-Methyl-2-butenyl, 2-Methyl-2-butenyl, 1-Methyl-3-butenyl, 2-Methyl-4-butenyl, 3-Methyl-3-butenyl, 1,1-Dimethyl-2-propenyl,1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-2-butenyl, 1,3-Dimethyl-3-butenyl, 2,2-Dimethyl-3-butenyl, 2,3-Dimethyl-2-butenyl, 2,3-Dimethyl-3-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, 1-Ethyl-1-methyl-2-propenyl und Ethyl-2-methyl-2-propenyl,
insbesondere 1-Methyl-2-propenyl, 1-Methyl-2-butenyl, 1,1-Dimethyl-2-propenyl und 1,1-Dimethyl-2-butenyl,
C₃-C₁₀-Alkinyl wie Propargyl, 2-Butinyl, 3-Butinyl, 2-Pentinyl, 3-Pentinyl, 4-Pentinyl, 1-Methyl-3-butinyl, 2-Methyl-3-butinyl, 1-Methyl-2-butinyl, 1,1-Dimethyl-2-propinyl, 1-Ethyl-2-propinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 3-Methyl-4-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 1-Ethyl-2-butinyl, 1-Ethyl-3-butinyl, 2-Ethyl-3-butinyl und 1-Ethyl-1-methyl-2-propinyl;
insbesondere Propargyl und 1,1-Dimethyl-2-propinyl;
C₃-C₈-Cycloalkyl wie Cyclopropyl, Cyclobutyl, Cyclopentyl, Cyclohexyl, Cycloheptyl, Cyclooctyl, insbesondere Cyclopropyl und Cyclohexyl;
wobei die o.g. organischen Gruppen ein bis fünf Halogenatome wie Fluor, Chlor, Brom und Iod, vorzugsweise Fluor, Chlor und Brom, insbesondere Fluor und Chlor,
und/oder einen bis drei der folgenden Reste tragen können:
Cyano;
C₃-C₈-Cycloalkyl wie Cyclopropyl, Cyclobutyl, Cyclopentyl, Cyclohexyl, Cycloheptyl, Cyclooctyl, insbesondere Cyclopropyl und Cyclohexyl;
C₁-C₄-Alkoxy wie Methoxy, Ethoxy, n-Propoxy, 2-Methylethoxy, n-Butoxy, 1-Methylpropoxy, 2-Methylpropoxy und 1,1-Dimethylethoxy, insbesondere C₁-C₃-Alkoxy wie Methoxy, Ethoxy, i-Propoxy;
C₁-C₄-Halogenalkoxy wie Chlormethoxy, Dichlormethoxy, Trichlormethoxy, Fluormethoxy, Difluormethoxy, Trifluormethoxy, Chlordifluormethoxy, Dichlorfluormethoxy, 1-Fluorethoxy, 2-Fluorethoxy, 2,2-Difluorethoxy, 1,1,2,2-Tetrafluorethoxy, 2,2,2-Trifluorethoxy, 2-Chlor-1,1,2-trifluorethoxy und Pentafluorethoxy, insbesondere C₁-C₃-Halogenalkoxy wie 2,2,2-Trifluorethyloxy und 2-Chlor-2,2-difluorethoxy;
C₁-C₄-Alkylthio wie Methylthio, Ethylthio, n-Propylthio, 1-Methylethylthio, n-Butylthio, 1-Methylpropylthio, 2-Methylpropylthio und 1,1-Dimethylethylthio, insbesondere Methylthio;
C₁-C₄-Halogenalkylthio wie Difluromethylthio, Trifluormethylthio, Chlordifluormethylthio, 1-Fluorethylthio, 2-Fluorethylthio, 2,2-Difluorethylthio, 2,2,2-Trifluorethylthio 2-Chlor-2,2-difluorethylthio, 2,2-Dichlor-2-fluorethylthio, 2,2,2-Trichlorethylthio und Pentafluorethylthio, insbesondere Trichlormethylthio;
Di-C₁-C₄-Alkylamino wie Dimethylamino, Diethylamino, Dipropylamino, Dibutylamino, N-Methyl-N-ethylamino, N-Methyl-N-propylamino, N-Methyl-N-1-methylethylamino, N-Methyl-N-1,1-Dimethylethylamino, Di-1-Methylethylamino, N-Ethyl-N-1-Methylethylamino und N-Ethyl-N-1,1-dimethyl ethylamino;
Phenyl, Phenylthio, Phenoxy, wobei die Phenylreste ihrerseits eine bis fünf Halogenatome wie Fluor, Chlor, Brom und Iod, insbesondere Fluor und Chlor,
und/oder eine bis drei der folgenden Gruppen tragen können:
Cyano oder Nitro;
C₁-C₄-Alkyl, wie vorstehend genannt, insbesondere Methyl, Ethyl, Propyl, 1-Methylpropyl und 1,1-Dimethylpropyl;
C₁-C₄-Alkoxy, wie vorstehend genannt, insbesondere Methoxy und Ethoxy;
C₁-C₄-Halogenalkyl, wie vorstehend genannt, insbesondere Trifluormethyl;
C₁-C₄-Halogenalkoxy, wie vorstehend genannt, insbesondere Trifluormethoxy;
- R²: Wasserstoff, Hydroxy;
C₁-C₈-Alkoxy, z. B. Methoxy, Ethoxy, n-Propoxy, 2-Methylethoxy, n-Butoxy, 1-Methylpropoxy, 2-Methylpropoxy und 1,1-Dimethylethoxy, insbesondere C₁-C₄-Alkoxy wie Methoxy, Ethoxy, Propoxy, Butoxy;
C₃-C₈-Alkenyloxy wie 2-Propenyloxy, 2-Butenyloxy, 3-Butenyloxy, 1-Methyl-2-propenyloxy, 2-Methyl-2-propenyloxy, 2-Pentenyloxy, 3-Pentenyloxy, 4-Pentenyloxy, 3-Methyl-2-butenyloxy, 1-Methyl-2-butenyloxy, 2-Methyl-2-butenyloxy, 1-Methyl-3-butenyloxy, 2-Methyl-3-butenyloxy, 3-Methyl-3-butenyloxy, 1,1-Dimethyl-2-propenyloxy, 1,2-Dimethyl-2-propenyloxy, 1-Ethyl-2-propenyloxy, 2-Hexenyloxy, 3-Hexenyloxy, 4-Hexenyloxy, 5-Hexenyloxy, 1-Methyl-2-pentenyloxy, 2-Methyl-2-pentenyloxy, 3-Methyl-2-pentenyloxy, 4-Methyl-2-pentenyloxy, 1-Methyl-3-pentenyloxy, 2-Methyl-3-pentenyloxy, 3-Methyl-3-pentenyloxy, 4-Methyl-4-pentenyloxy, 1-Methyl-4-pentenyloxy, 2-Methyl-4-pentenyloxy, 3-Methyl-4-pentenyloxy, 4-Methyl-4-pentenyloxy, 1,1-Dimethyl-2-butenyloxy, 1,1-Dimethyl-3-butenyloxy, 1,2-Dimethyl-2-butenyloxy, 1,2-Dimethyl-3-butenyloxy, 1,3-Dimethyl-2-butenyloxy, 1,3-Dimethyl-3-butenyloxy, 2,2-Dimethyl-3-butenyloxy, 2,3-Dimethyl-2-butenyloxy, 2,3-Dimethyl-4-butenyloxy, 1-Ethyl-2-butenyloxy, 1-Ethyl-3-butenyloxy, 2-Ethyl-2-butenyloxy, 2-Ethyl-3-butenyloxy, 1,1,2-Trimethyl-2-propenyloxy, 1-Ethyl-1-methyl-2-propenyloxy und Ethyl-2-methyl-2-propenyloxy, insbesondere 2-Propenyloxy; Di-C₁-C₄-Alkylamino, C₁-C₁₀-Alkyl, C₃-C₁₀-Alkenyl und C₃-C₁₀-Alkinyl sowie C₃-C₈-Cycloalkyl₁ jeweils wie vorstehend für R1 im allgemeinen und im besonderen genannt,
wobei die genannten organischen Gruppen ein bis fünf Halogenatome wie Fluor, Chlor, Brom und Iod, vorzugsweise Fluor, Chlor und Brom, insbesondere Fluor und Chlor,
und/oder einen bis drei der folgenden Reste tragen können:
Cyano,
C₃-C₈-Cycloalkyl, C₁-C₄-Alkoxy, C₁-C₄-Halogenalkoxy, C₁-C₄-Alkylthio, C₁-C₄-Halogenalkylthio und Di-C₁-C₄-Alkylamino, jeweils wie vorstehend im einzelnen angegeben,
Phenyl, Phenylthio, Phenoxy, wobei die Phenylreste ihrerseits eine bis fünf Halogenatome wie Fluor, Chlor, Brom und Iod, insbesondere Fluor und Chlor,
und/oder eine bis drei der folgenden Gruppen tragen können:
Cyano oder Nitro,
C₁-C₄-Alkyl, C₁-C₄-Alkoxy, C₁-C₄-Halogenalkyl und C₁-C₄-Halogenalkoxy, jeweils wie vorstehend genannt;
- R²: ferner substituiertes C₁-C₈-Alkoxy, C₃-C₈-Alkenyloxy, Di-C₁-C₄-Alkylamino, C₁-C₁₀-Alkyl, C₃-C₁₀-Alkenyl, C₃-C₁₀-Alkinyl oder C₃-C₈-Cycloalkyl, wobei diese Gruppen durch ein bis fünf Halogenatome und/oder ein bis zwei 5- bis 6-gliedrige heterocyclische, aliphatische oder aromatische Reste, enthaltend ein bis drei Heteroatome, ausgewählt aus der Gruppe Sauerstoff, Schwefel oder Stickstoff, substituiert sind; beispielsweise seien folgende heterocyclische Reste genannt: 2-Tetrahydrofuranyl, 3-Tetrahydrofuranyl, 2-Tetrahydothienyl, 3-Tetrahydrothienyl, 2-Tetrahydropyranyl, 3-Tetrahydropyranyl, 4-Tetrahydropyranyl, 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl, 1,3,4-Triazol-2-yl, 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl und 1,2,4-Triazin-3-yl;
wobei diese heterocyclischen Reste einen oder zwei der folgenden Substituenten tragen können:
Halogen wie Fluor, Chlor, Brom und Jod, insbesondere Fluor oder Chlor, C₁-C₄-Alkyl, C₁-C₄-Alkoxy, C₁-C₄-Halogenalkyl oder C₁-C₄-Halogenalkoxy wie vorstehend genannt;
R¹ und R² gemeinsam eine Alkylenkette mit 4 bis 7 Gliedern, welche durch Sauerstoff, Schwefel oder N-Methyl unterbrochen sein kann wie -(CH₂)₄-, -(CH₂)₅-, -(CH₂)₆-, -CH₂-CH₂-O-CH₂-CH₂-, -CH₂-CH₂-S-CH₂-CH₂-, -CH₂-CH₂-(NCH₃)-CH₂-CH₂-, insbesondere -(CH₂)₅- und - CH₂-CH₂-O-CH₂-CH₂-;
- R³: Wasserstoff;
Halogen wie Fluor, Chlor, Brom und Jod, insbesondere Fluor oder Chlor;
Nitro;
C₁-C₁₀-Alkyl, C₂-C₁₀-Alkenyl, C₂-C₁₀-Alkinyl, C₃-C₈-Cycloalkyl, C₁-C₆-Alkoxy, C₁-C₆-Halogenalkoxy, C₁-C₆-Alkylthio, C₁-C₆-Haloenalkylthio, Di-C₁-C₆-Alkylamino, jeweils wie vorstehend für R¹ im einzelnen genannt;
C₇-C₁₅-Arylalkyl, insbesondere Phenyl-C₁-C₄-alkyl wie Benzyl, 1-Phenylethyl, 2-Phenylethyl, 1-Phenylpropyl, 2-Phenylpropyl, 3-Phenylpropyl, 1-Methyl-1-phenylethyl, 1-Methyl-2-phenylethyl, 1-Phenylbutyl, 2-Phenylbutyl, 3-Phenylbutyl, 4-Phenylbutyl, besonders bevorzugt Benzyl;
Phenyl, Phenylthio, Phenoxy oder ein 5- oder 6-gliedriger heterocyclischer, aliphatischer oder aromatischer Rest, enthaltend ein bis drei Heteroatome, ausgewählt aus der Gruppe Sauerstoff, Schwefel oder Stickstoff, wie vorstehend für R² im einzelnen genannt, wobei die genannten aromatischen oder heteroaromatischen Reste gegebenenfalls substituiert sind durch Halogen, Cyano, Nitro, C₁-C₄-Alkyl, C₁-C₄-Halogenalkyl, C₃-C₈-Cycloalkyl, C₁-C₄-Alkoxy, C₁-C₄-Halogenalkoxy, C₁-C₄-Alkylthio, C₁-C₄-Halogenalkylthio oder Di-C₁-C₄-Alkylamino, jeweils wie vorstehend für R¹ im einzelnen genannt.

Beispiele für besonders bevorzugte Verbindungen der allgemeinen Formel I sind in der folgenden Tabelle zusammengestellt. Die darin für einen Substituenten genannten Gruppen stellen außerdem für sich betrachtet - unabhängig von der speziellen Kombination mit anderen Substituenten, in der sie genannt sind - eine besonders bevorzugte Definition des betreffenden Substituenten dar.

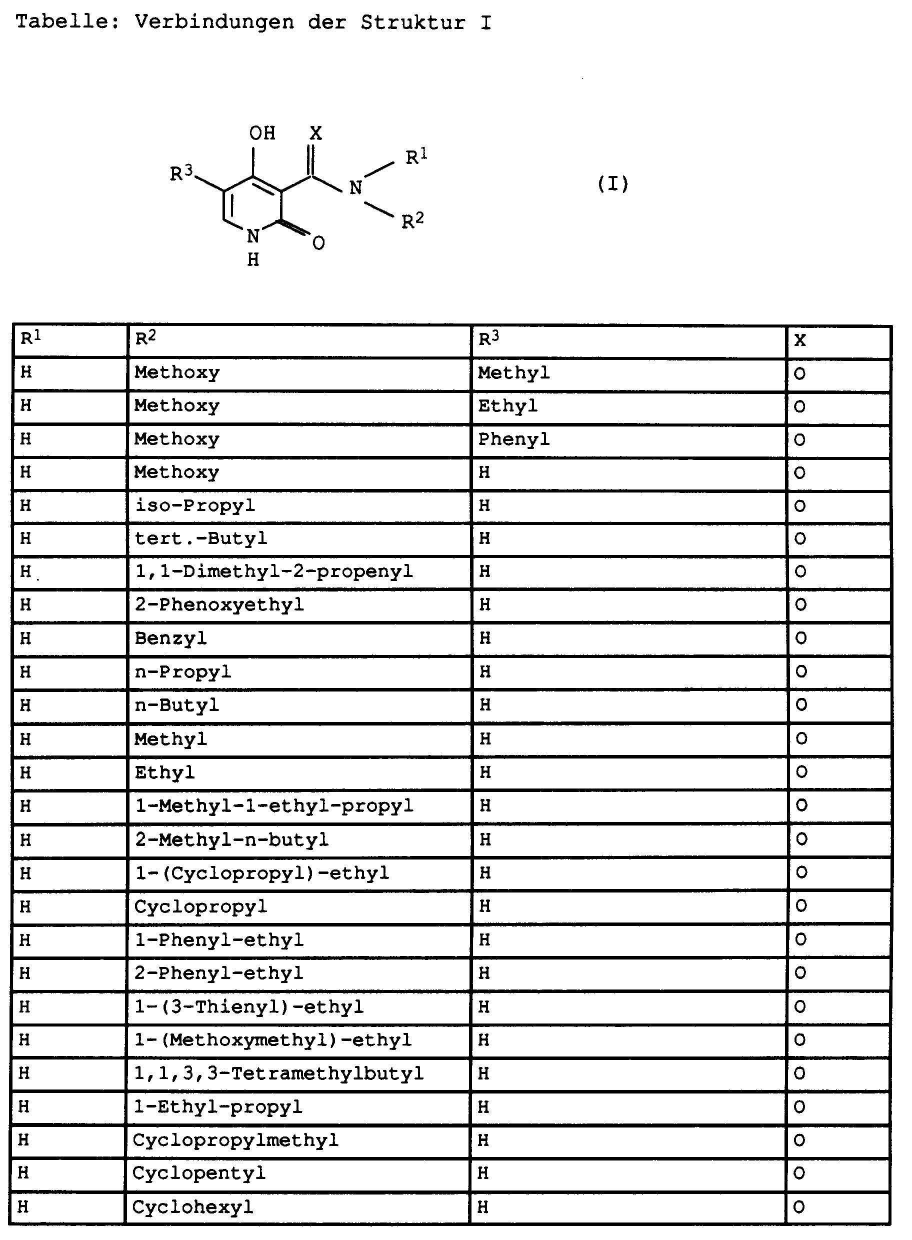

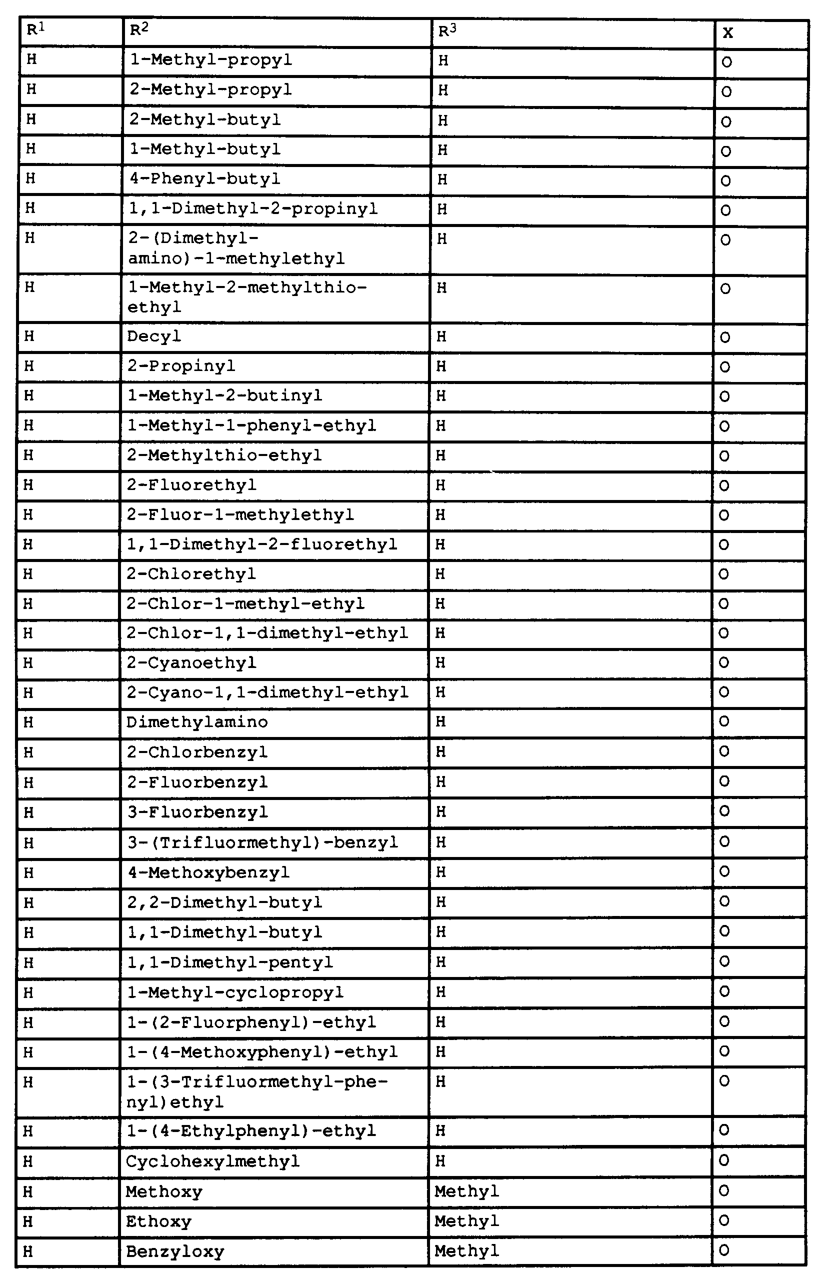

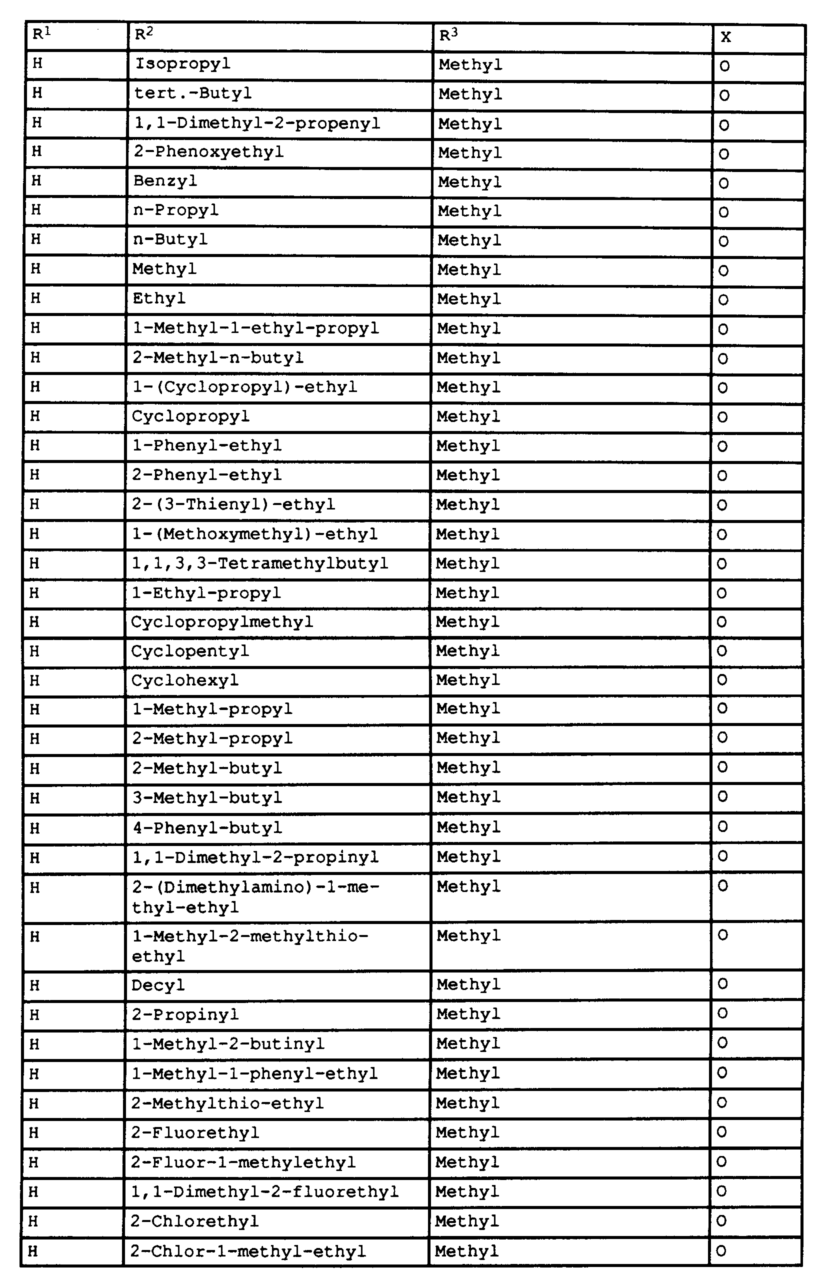

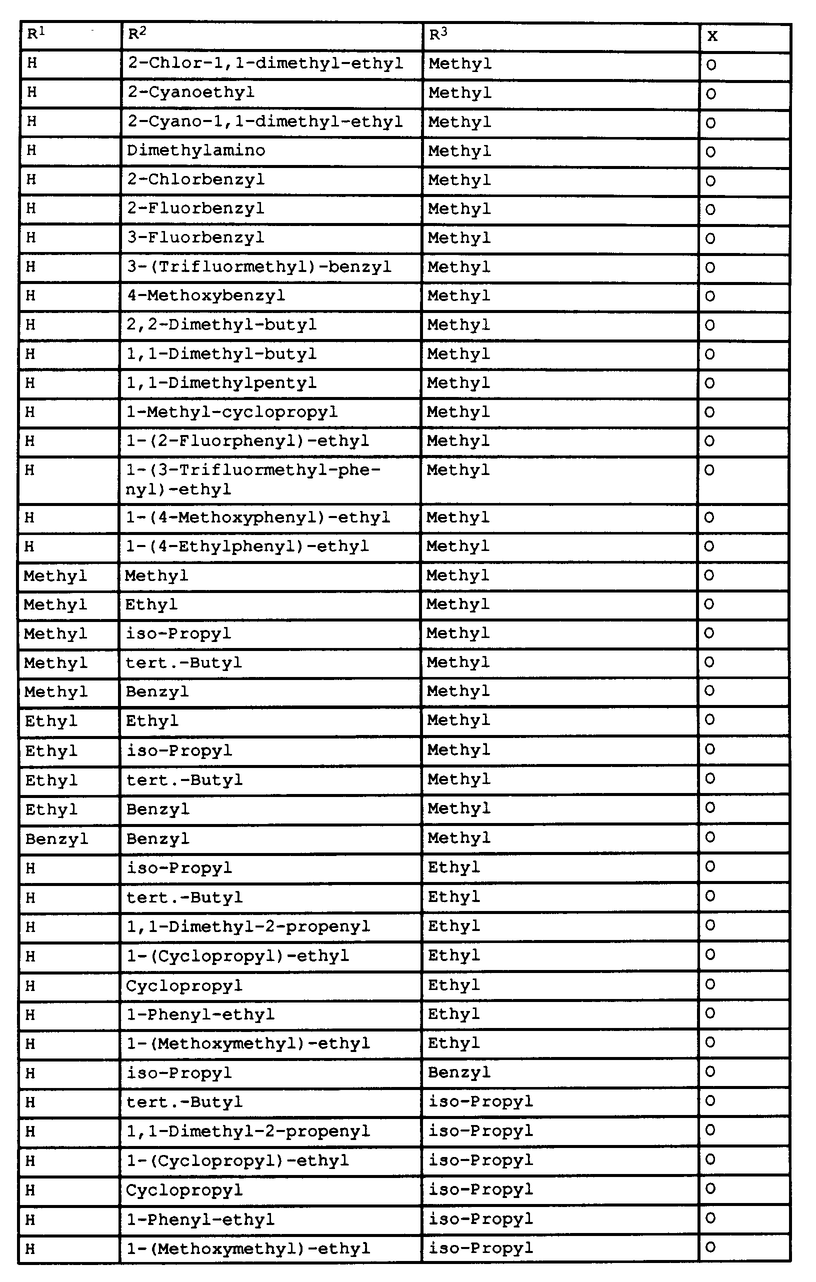

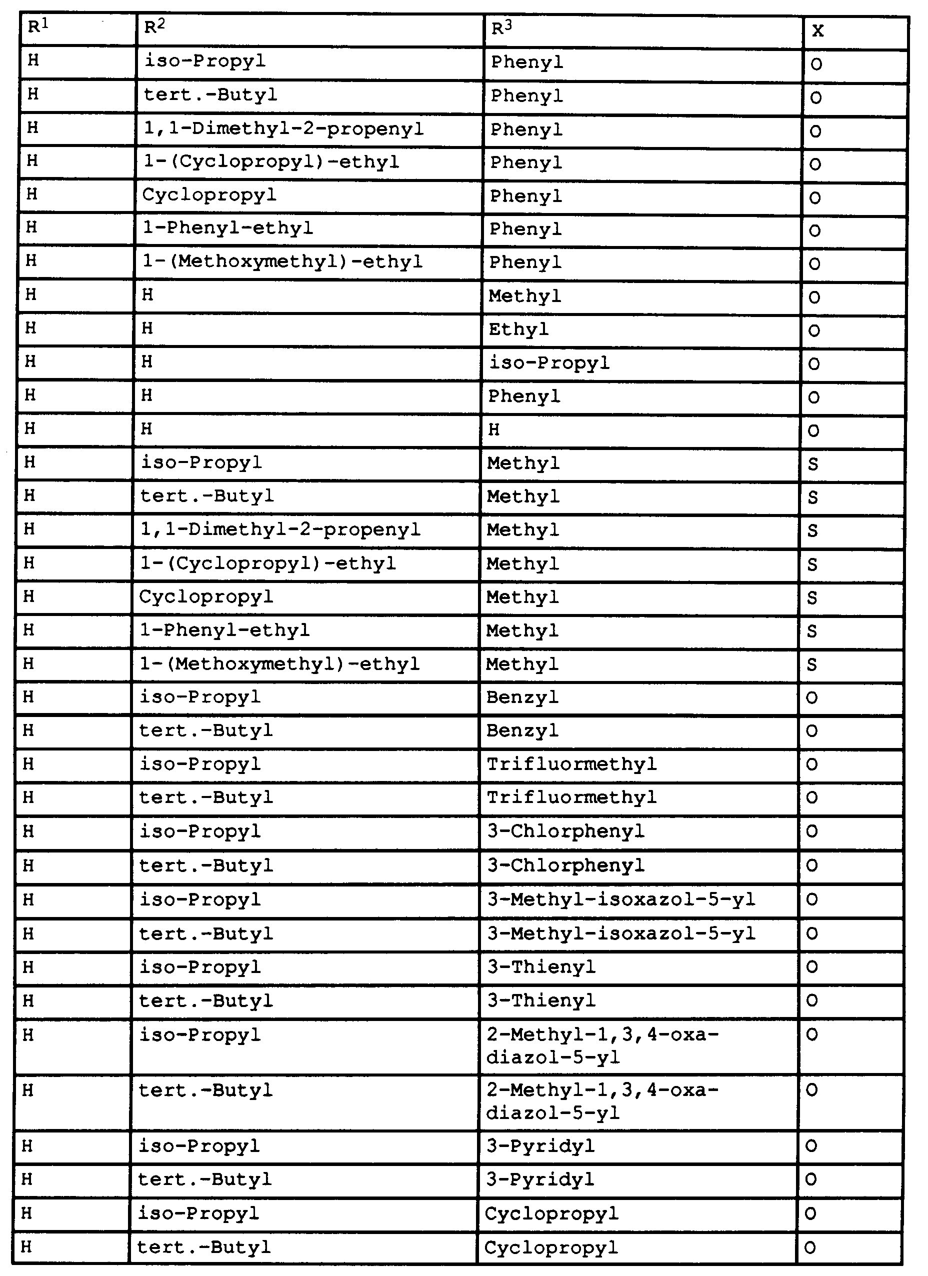

Die Verbindungen I bzw. die sie enthaltenden herbiziden Mittel sowie deren umweltverträgliche Salze von beispielsweise Alkalimetallen, Erdalkalimetallen oder Aminen bzw. die sie enthaltenden herbiziden Mittel können in Kulturen wie Weizen, Reis, Mais, Soja und Baumwolle Unkräuter und Schadgräser sehr gut bekämpfen, ohne die Kulturpflanzen nennenswert zu schädigen. Dieser Effekt tritt vor allem bei niedrigen Aufwandmengen auf.

Unter Berücksichtigung der Vielseitigkeit der Applikationsmethoden können die Verbindungen I bzw. sie enthaltende Mittel noch in einer weiteren Zahl von Kulturpflanzen zur Beseitigung unerwünschter Pflanzen eingesetzt werden. In Betracht kommen beispielsweise folgende Kulturen:
Allium cepa, Ananas comosus, Arachis hypogaea, Asparagus officinalis, Beta vulgaris spp. altissima, Beta vulgaris spp. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spp., Manihot esculenta, Medicago sativa, Musa spp., Nicotiana tabacum (N.rustica), Olea europaea, Oryza sativa, Phaseolus lunatus, Phaseolus vulgaris, Picea abies, Pinus spp., Pisum sativum, Prunus avium, Prunus persica, Pyrus communis, Ribes sylvestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera, Zea mays.

Darüber lassen sich die Verbindungen I auch in Kulturen, die durch Züchtung und/oder Anwendung gentechnischer Methoden gegen die Wirkung von I oder anderen Herbiziden tolerant sind, einsetzen.

Die Applikation der herbiziden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Verbindungen I bzw. die sie enthaltenden herbiziden Mittel können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Als inerte Hilfsstoffe für die Herstellung von direkt versprühbaren Lösungen, Emulsionen, Pasten oder Öldispersionen kommen im wesentlichen in Betracht: Mineralölfraktionen von mittlerem bis hohem Siedepunkt wie Kerosin oder Dieselöl, ferner Kohlenteeröle sowie Öle pflanzlichen oder tierischen Ursprungs, aliphatische, cyclische und aromatische Kohlenwasserstoffe, z.B. Paraffine, Tetrahydronaphthalin, alkylierte Naphthaline oder deren Derivate, alkylierte Benzole und deren Derivate, Alkohole wie Methanol, Ethanol, Propanol, Butanol und Cyclohexanol, Ketone wie Cyclohexanon oder stark polare Lösungsmittel, z.B. Amine wie N-Methylpyrrolidon, oder Wasser.

Wäßrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Formulierungen enthalten im allgemeinen zwischen 0,01 und 95 Gew.-%, vorzugsweise zwischen 0,5 und 90 Gew.-%, Wirkstoff. Die Wirkstoffe werden dabei in einer Reinheit von 90 % bis 100 %, vorzugsweise 95 % bis 100 % (nach NMR-Spektrum) eingesetzt. Die erfindungsgemäßen Verbindungen I können beispielsweise wie folgt formuliert werden:
I. 20 Gewichtsteile der Verbindung Nr. 2.006 werden in einer Mischung gelöst, die aus 80 Gewichtsteilen alkyliertem Benzol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew. % des Wirkstoffs enthält.
II. 20 Gewichtsteile der Verbindung Nr. 2.006 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.-% des Wirkstoffs enthält.
III. 20 Gewichtsteile des Wirkstoffs Nr. 2.006 werden in einer Mischung gelöst, die aus 25 Gewichtsteilen Cyclohexanon, 65 Gewichtsteilen einer Mineralölfraktion vom Siedepunkt 210 bis 280C° und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.-% des Wirkstoffs enthält.
IV. 20 Gewichtsteile des Wirkstoffs Nr. 2.006 werden mit 3 Gewichtsteilen des Natriumsalzes der Diisobutylnaphthalin-α-sulfonsäure, 17 Gewicht steilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser enthält man eine Spritzbrühe, die 0,1 Gew.-% des Wirkstoffs enthält.
V. 3 Gewichtsteile des Wirkstoffs Nr. 2.006 werden mit 97 Gewichtsteilen feinteiligem Kaolin vermischt. Man erhält auf diese Weise ein Stäubemittel, das 3 Gew.-% des Wirkstoffs enthält.
VI. 20 Gewichtsteile des Wirkstoffs Nr. 2.006 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.

Zur Verbreiterung des Wirkungsspektrums und zur Erzielung synergistischer Effekte können die Hydroxypyridoncarbonsäureamide I mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, die in 2-Stellung z.B. eine Carboxy- oder Carbimino-Gruppe tragen, Chinolincarbonsäurederivate, Imidazolinone, Sulfonamide, Sulfonylharnstoffe, Aryloxy-, Heteroaryloxyphenoxypropionsäuren sowie deren Salze, Ester und Amide und andere in Betracht.

Außerdem kann es von Nutzen sein, die Verbindungen I allein oder in Kombination mit anderen Herbiziden auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

Die Aufwandmengen an Wirkstoff betragen je nach Bekämpfungsziel, Jahreszeit, Zielpflanzen und Wachstumsstadium 0,001 bis 3,0, vorzugsweise 0,01 bis 1,0 kg/ha aktive Substanz (a.S.).

### Synthesebeispiele

### Beispiel 1

### Herstellung der Ausgangsverbindungen

### Herstellung von 5-Ethyl-4-hydroxy-pyrid-2-on-3-carbonsäureethylester

4,2 g (0,17 mol) Natriumhydrid werden in 500 ml Toluol suspendiert und tropfenweise mit 27 g (0,17 mol) Diethylmalonat versetzt. Die Suspension wird 2 Stunden bei Raumtemperatur gerührt und anschließend zu einer Lösung von 16 g (0,17 mol) 1-Butenylisocyanat in 300ml Toluol getropft. Das Reaktionsgemisch wird über Nacht bei Raumtemperatur gerührt und dann mit Ammoniumchlorid-Lösung auf pH 6 eingestellt. Die organische Phase wird abgetrennt, mit Natriumsulfat getrocknet und eingeengt. Der Rückstand wird aus Cyclohexan umkristallisiert. Man erhält 32 g weiße Kristalle (Schmp.: 85 °C).

15 g dieser Kristalle werden für 3 Minuten auf 200 °C erhitzt. Der verbleibende braune Rückstand wird säulenchromatographisch über Kieselgel aufgetrennt. Nach Umkristallisation aus Diisopropylether erhält man 2,4 g 5-Ethyl-4-hydroxy-pyrid-2-on-3-carbonsäure-ethylester (Schmp.: 116 - 117 °C).

In entsprechender Weise wurden unter Abwandlung der Ausgangsstoffe weitere Vorprodukte IIa hergestellt. Die so erhaltenen Verbindungen sind in der nachstehenden Tabelle 1 mit physikalischen Angaben aufgeführt.

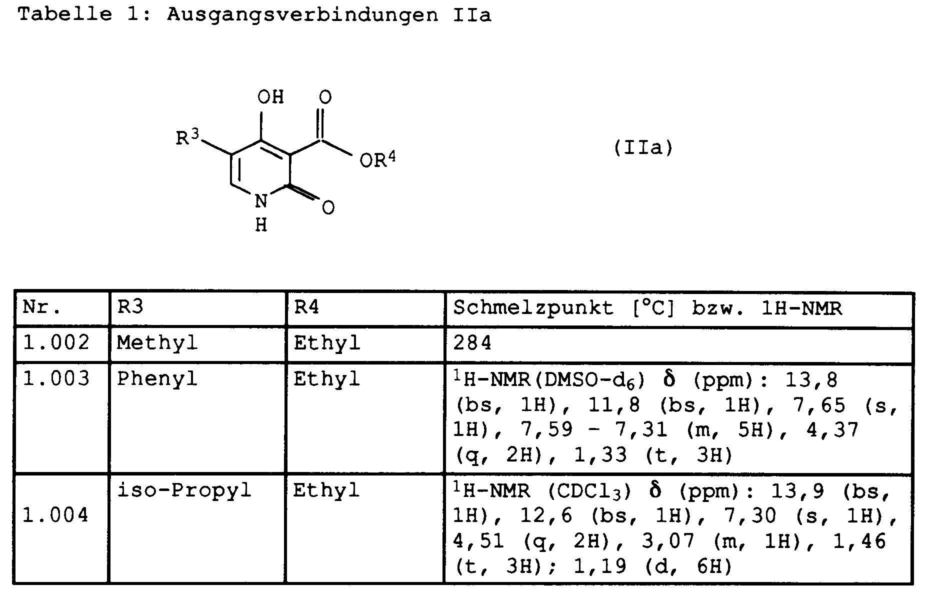

### Beispiel 2

### Herstellung von 5-Ethyl-4-hydroxy-pyrid-2-on-3-carbonsäure-isopropylamid (Nr. 2.002 der Tabelle 2)

1,9 g (9 mmol) 5-Ethyl-4-hydroxy-pyrid-2-on-3-carbonsäure-ethylester (1a) werden mit 0,6 g (9 mmol) Isopropylamin in 30 ml Ethanol in einem Miniautoklaven 8 Stunden bei 150 °C unter Eigendruck gerührt. Nach dem Abkühlen wird der Rückstand abfiltriert, die Mutterlauge eingeengt und säulenchromatographisch über Kieselgel gereinigt.
Ausbeute: 0,75 g (37 % der Theorie), Fp.: 117 °C.
In entsprechender Weise wurden unter Abwandlung der Ausgangsstoffe weitere Verbindungen I hergestellt. Die so erhaltenen Verbindungen sind in der nachstehenden Tabelle 2 mit physikalischen Angaben aufgeführt.

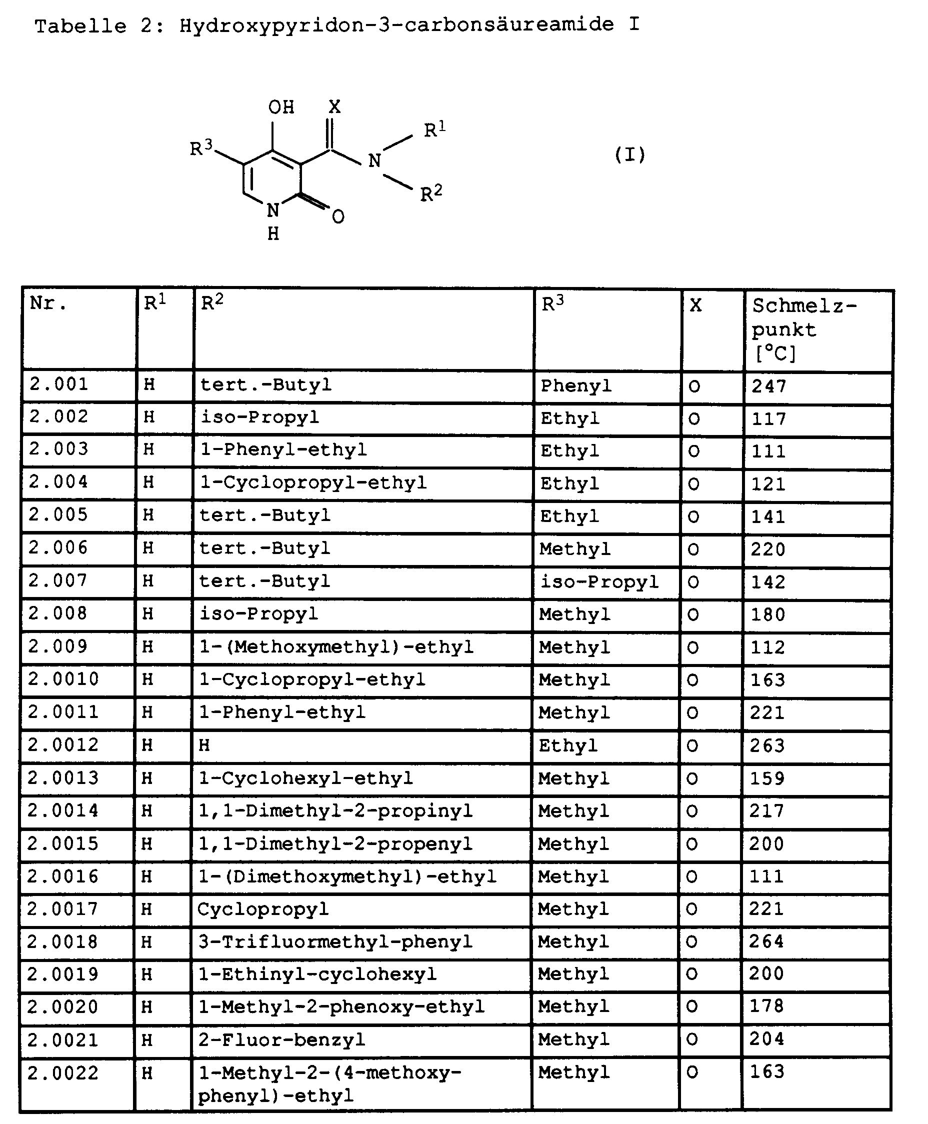

### Beispiel 3

### Herstellung von 5-Methyl-4-hydroxy-pyrid-2-on-3-carbonsäure-tert.-butylamid, Na-Salz (Nr. 3.002 der Tabelle 3)

0,63 g (2,8 mmol) 5-Methyl-4-hydroxy-pyrid-2-on-3-carbonsäure-tert.-butylamid werden in Methanol suspendiert und mit einer Lösung von 150 mg (2,8 mmol) Natriummethylat in Methanol versetzt. Die Lösung wird eine Stunde bei Raumtemperatur gerührt, anschließend eingeengt und der Rückstand getrocknet.
Ausbeute: 0,68 g (98 % der Theorie), Fp.. 280 °C
In entsprechender Weise wurden unter Abwandlung der Ausgangsstoffe weitere Salze von Verbindungen I hergestellt. Die so erhaltenen Salze sind in der nachstehenden Tabelle 3 mit physikalischen Angaben aufgeführt.

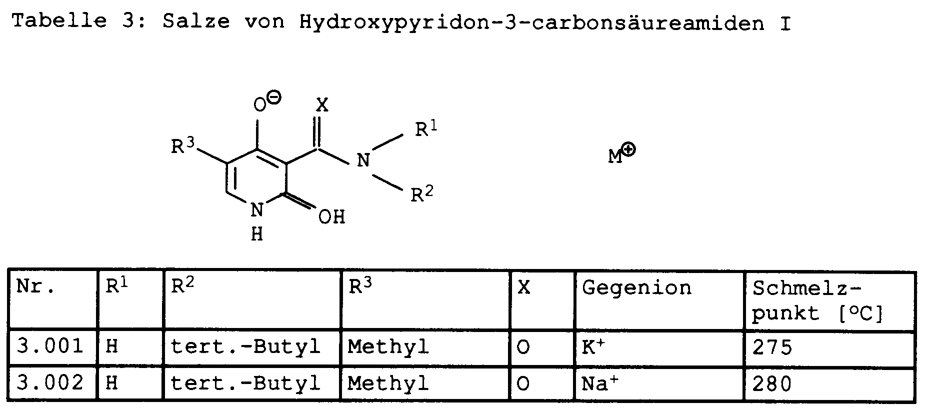

### Anwendungsbeispiele

Die herbizide Wirkung der Hydroxypyridoncarbonsäureamide der Formel I ließ sich durch Gewächshausversuche zeigen:

Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zweck der Nachauflaufbehandlung wurden die Testpflanzen je nach Wuchsform erst bis zu einer Wuchshöhe von 3 bis 15 cm angezogen und erst dann mit den in Wasser suspendierten oder emulgierten Wirkstoffen behandelt. Die Testpflanzen wurden dafür entweder direkt gesät und in den gleichen Gefäßen aufgezogen oder sie werden erst als Keimpflanzen getrennt angezogen und einige Tage vor der Behandlung in die Versuchsgefäße verpflanzt. Die Aufwandmenge für die Nachauflaufbehandlung betrug 0,5 bzw. 0,25 kg/ha a.S.

Die Pflanzen wurden artenspezifisch bei Temperaturen von 10 - 25°C bzw. 20 - 35C° gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Abkürzung | Lateinischer Name | Deutscher Name |
|---|---|---|
| ECHCG | Echinochloa crus-galli | Hühnerhirse |
| ABUTH | Abutilon theophrasti | Chinesischer Hanf |
| POLPE | Polygonum persicaria | Flohknöterich |
| SINAL | Sinapis alba | Weißer Senf |

Das Ergebnis zeigte, daß mit Verbindung Nr. 2.006 unerwünschte Pflanzen sehr gut bekämpft werden können.

Im Vergleich zur strukturell nächstliegenden Verbindung des Standes der Technik, der aus EP-A 544 151 bekannten Vergleichssubstanz A 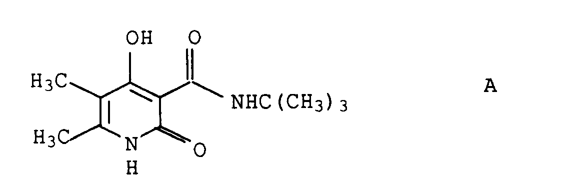
weist Verbindung Nr. 2.006, insbesondere bei niedrigen Aufwandmengen von 0,25 kg/ha eine bessere herbizide Wirkung auf, wie die nachstehend in Tabelle I zusammengefaßten Ergebnisse zeigen.

**Tabelle I**

| Gegenüberstellung von Ergebnissen aus Gewächshausversuchen im Nachauflaufverfahren | | | | |
|---|---|---|---|---|
| Beispiel Nr. | 2.006 | | A | |
| Aufwandmenge (kg/ha s.A.) | 0,5 | 0,25 | 0,5 | 0,25 |
| Testpflanzen (Schädigung in %) | | | | |
| ECHCG | 90 | 90 | 90 | 40 |
| ABUTH | 100 | 100 | 85 | 70 |
| POLPE | 100 | 100 | 70 | 10 |
| SINAL | 100 | 100 | 100 | 80 |

## Patentansprüche

1. Hydroxypyridoncarbonsäureamide der allgemeinen Formel I 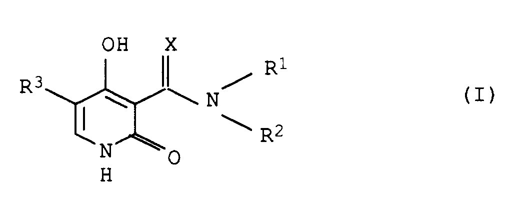 in der die Substituenten folgende Bedeutungen haben:
X Sauerstoff oder Schwefel;
R¹ Wasserstoff, C₁-C₁₀-Alkyl, C₃-C₁₀-Alkenyl, C₃-C₁₀-Alkinyl, C₃-C₈-Cycloalkyl, Phenyl, wobei diese Gruppen ein bis fünf Halogenatome und/oder einen bis drei der folgenden Reste tragen können: Cyano, C₃-C₈-Cycloalkyl, C₁-C₄-Alkoxy, C₁-C₄-Halogenalkoxy, C₁-C₄-Alkylthio, C₁-C₄-Halogenalkylthio, Di-C₁-C₄-Alkylamino, Phenyl, Phenylthio, Phenoxy, wobei die Phenylreste ihrerseits eine bis drei der folgenden Gruppen tragen können: C₁-C₄-Alkyl, C₁-C₄-Alkoxy, C₁-C₄-Halogenalkyl,C₁-C₄-Halogenalkoxy, Halogen, Cyano oder Nitro;
R² Wasserstoff, Hydroxyl, C₁-C₈-Alkoxy, C₃-C₈-Alkenyloxy, Di-C₁-C₄-Alkylamino, C₁-C₁₀-Alkyl, C₃-C₁₀-Alkenyl, C₃-C₁₀-Alkinyl, C₃-C₈-Cycloalkyl, wobei die Gruppen ein bis fünf Halogenatome und/oder einen bis drei der folgenden Reste tragen können: Cyano, C₃-C₈-Cycloalkyl, C₁-C₄-Alkoxy, C₁-C₄-Halogenalkoxy, C₁-C₄-Alkylthio, C₁-C₄-Halogenalkylthio, Di-C₁-C₄-Alkylamino, Phenyl, Phenylthio, Phenoxy, wobei die Phenylreste ihrerseits eine bis drei der folgenden Gruppen tragen können: C₁-C₄-Alkyl, C₁-C₄-Alkoxy, C₁-C₄-Halogenalkyl, C₁-C₄-Halogenalkoxy, Halogen, Cyano oder Nitro;
R² ferner substituiertes C₁-C₈-Alkoxy, C₃-C₈-Alkenyloxy, Di-C₁-C₄-Alkylamino, C₁-C₁₀-Alkyl, C₃-C₁₀-Alkenyl, C₃-C₁₀-Alkinyl, C₃-C₈-Cycloalkyl, wobei diese Gruppen durch ein bis fünf Halogenatome und/oder ein bis zwei 5- bis 6-gliedrige heteroyclische, aliphatische oder aromatische Reste, enthaltend ein bis drei Heteroatome, ausgewählt aus der Gruppe Sauerstoff, Schwefel oder Stickstoff substituiert sind, die ein oder zwei der folgenden Substituenten tragen können: Halogen, C₁-C₄-Alkyl, C₁-C₄-Alkoxy, C₁-C₄-Halogenalkyl oder C₁-C₄-Halogenalkoxy;
oder
R¹, R² gemeinsam eine Alkylenkette mit 4 bis 7 Gliedern, welche durch Sauerstoff, Schwefel oder N-Methyl unterbrochen sein kann;
R³ Wasserstoff, Halogen, Nitro, C₁-C₁₀-Alkyl, C₂-C₁₀-Alkenyl, C₂-C₁₀-Alkinyl, C₃-C₈-Cycloalkyl, C₇-C₁₅-Arylalkyl, C₁-C₆-Alkoxy, C₁-C₆-Halogenalkoxy, C₁-C₆-Alkylthio, C₁-C₆-Halogenalkylthio, Di-C₁-C₆-Alkylamino, Phenyl, Phenylthio, Phenoxy oder ein 5- oder 6-gliedriger heterocyclischer, aliphatischer oder aromatischer Rest, enthaltend ein bis drei Heteroatome, ausgewählt aus der Gruppe Sauerstoff, Schwefel oder Stickstoff, wobei die genannten aromatischen oder heteroaromatischen Reste gegebenenfalls substituiert sind durch Halogen, Cyano, Nitro, C₁-C₄-Alkyl, C₁-C₄-Halogenalkyl, C₃-C₈-Cycloalkyl, C₁-C₄-Alkoxy, C₁-C₄-Halogenalkoxy, C₁-C₄-Alkylthio, C₁-C₄-Halogenalkylthio oder Di-C₁-C₄-Alkylamino;
und deren umweltverträgliche Salze.

2. Hydroxypyridoncarbonsäureamide der Formel I gemäß Anspruch 1, in der X und die Reste R¹ bis R³ folgende Bedeutung haben:
X Sauerstoff;
R¹ Wasserstoff;
R² Wasserstoff, C₁-C₁₀-Alkyl, C₃-C₁₀-Alkenyl, C₃-C₁₀-Alkinyl, C₃-C₈-Cycloalkyl, C₁-C₈-Alkoxy, wobei die genannten Reste durch Halogen, C₁-C₄-Alkoxy, C₁-C₄-Halogenalkoxy oder Phenyl substituiert sein können, wobei der Phenylrest seinerseits ein bis drei der folgenden Gruppen tragen kann:
C₁-C₄-Alkyl, C₁-C₄-Alkoxy, C₁-C₄-Halogenalkyl oder C₁-C₄-Halogenalkoxy;
R³ Wasserstoff, C₁-C₁₀-Alkyl, C₁-C₁₀-Halogenalkyl, C₂-C₁₀-Alkenyl, C₂-C₁₀-Halogenalkenyl, C₂-C₁₀-Alkinyl, C₃-C₁₀-Cycloalkyl, C₇-C₁₅-Arylalkyl oder Halogen
und deren umweltverträgliche Salze.

3. Verfahren zur Herstellung der Hydroxypyridoncarbonsäureamide der Formel I gemäß Anspruch 1, dadurch gekennzeichnet, daß man Verbindungen der Formel II, 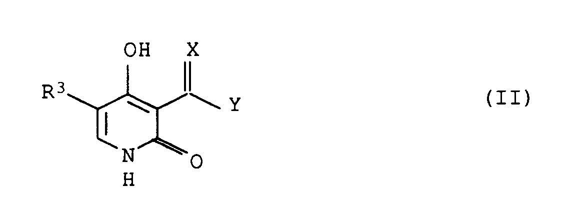 in der X und R³ die in Anspruch 1 angegebene Bedeutungen haben und Y ein Halogenatom, eine OH-Gruppe oder eine Alkoxygruppe bedeuten, mit einem Amin der Formel III, 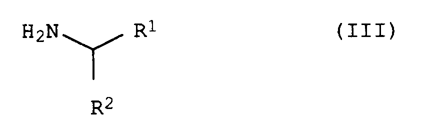 in der R¹ und R² die in Anspruch 1 angegebenen Bedeutungen haben, unter üblichen Amidierungsbedingungen umsetzt.

4. Verfahren zur Bekämpfung unerwünschten Pflanzenwuchses, dadurch gekennzeichnet, daß man eine herbizid wirksame Menge mindestens eines Hydroxypyridoncarbonsäureamids der Formel I gemäß Anspruch 1 auf Pflanzen, deren Lebensraum oder auf Saatgut einwirken läßt.

5. Herbizides Mittel, enthaltend flüssige oder feste Trägerstoffe und eine herbizid wirksame Menge mindestens eines Hydroxypyridoncarbonsäureamids der Formel I gemäß Anspruch 1.

6. Verwendung der Hydroxypyridoncarbonsäureamide der Formel I gemäß Anspruch 1 als Herbizide oder zur Regulierung des Pflanzenwachstums.
